# EUROPÄISCHE PATENTSCHRIFT

(11) **EP 3 302 714 B1**
(45) Veröffentlichungstag und Bekanntmachung des Hinweises auf die Patenterteilung: **30.01.2019**
(21) Anmeldenummer: 16722895.6
(22) Anmeldetag: 17.05.2016
(51) Int. Cl.: A61Q 5/02, A61K 8/60

(54) **KOMBINATION VON ISOSORBIDDIESTERN MIT NICHTIONISCHEN TENSIDEN ALS PERLGLANZMITTEL**
COMBINATION OF ISOSORBIDE DIESTERS HAVING NON-IONIC SURFACTANTS AS PEARLESCENT AGENT
COMBINAISON DE DIESTERS D'ISOSORBIDE ET DE TENSIOACTIFS NON IONIQUES EN TANT QU'AGENT NACRÉ

(30) Priorität: 27.05.2015 EP 15169332
(43) Veröffentlichungstag der Anmeldung: 11.04.2018
(73) Patentinhaber: BASF SE, 67056 Ludwigshafen am Rhein (DE)
(72) Erfinder: STOER, Claudia, 40597 Düsseldorf (DE); WEISSENEGGER, Markus, 40627 Düsseldorf (DE); NIEENDICK, Claus, 47807 Krefeld (DE); WINZEK, Mirella, 52445 Titz (DE)
(74) Vertreter: BASF IP Association
(86) Internationale Anmeldenummer: PCT/EP2016/060959
(87) Internationale Veröffentlichungsnummer: WO 2016/188789

(56) Entgegenhaltungen:
- EP-A1- 2 239 315
- WO-A1-03/052037
- WO-A1-2013/041388
- US-A1- 2014 323 592

## Beschreibung

Die vorliegende Erfindung betrifft Zusammensetzungen, enthaltend mindestens einen Isosorbiddiester einer gesättigten Fettsäure und mindestens ein nichtionisches Tensid sowie Verfahren zu deren Herstellung. Weiterer Gegenstand der vorliegenden Erfindung ist die Verwendung entsprechender Zusammensetzungen als Perlglanzmittel in kosmetischen Zusammensetzungen.

Perlglanzmittel werden in kosmetischen Zusammensetzungen häufig verwendet, um die Ästhetik entsprechender Zubereitungen zu verbessern und ihnen eine besonders pflegende Erscheinung zu verleihen. Um die hohen Anforderungen des Marktes bezüglich sensorischer Eigenschaften zu erfüllen, werden daher kontinuierlich neue Perlglanzmittel entwickelt und deren Eignung in kosmetischen Zusammensetzungen getestet.

Die derzeit kommerziell erhältlichen bzw. beschriebenen Perlglanzmittel sind bei ihrer Verwendung in kosmetischen Zusammensetzungen noch nicht zufriedenstellend und es besteht daher weiterhin Bedarf an der Bereitstellung neuer Inhaltsstoffe, die zum Einsatz in kosmetischen Mitteln als Perlglanzmittel geeignet sind.

Der Stand der Technik kennt eine Vielzahl von Formulierungen, die oberflächenaktiven Reinigungsmitteln den gewünschten Perlglanz verleihen. So lassen sich beispielsweise gemäß EP-B1 0 569 843 nichtionische, fließfähige Perlglanzdispersionen erhalten, indem man Mischungen aus 5 bis 30 Gew.-% acylierten Polyglykolen und 0,1 bis 20 Gew.-% ausgewählten nichtionischen Tensiden herstellt. Aus der EP-A2 0 581 193 sind ferner fließfähige, konservierungsmittelfreie Perlglanzdispersionen bekannt, die acylierte Polyglykolether, Betaine, Aniontenside und Glycerin enthalten.

Aus der amerikanischen Patentanmeldungen US 2014/0323592 ist die Verwendung von Isosorbiddicaprylat als Verdicker in kosmetischen Mitteln wie Shampoos bekannt.

Trotz der Vielzahl von Mitteln besteht im Markt ein ständiges Bedürfnis nach neuen Perlglanzwachsen, insbesondere an Perlglanzwachsen, die möglichst keine Ethylenoxid- und/oder Propylenoxid-Einheiten aufweisen, da diese breit sowohl in klassischen als auch in sogenannten "grünen" Reinigungsmitteln mit Tensiden ohne Ethylenoxideinheiten einsetzbar sind. Dennoch wird von den neuen Perlglanzwachsen erwartet, dass sie ein vergleichbares Leistungsspektrum hinsichtlich Perlglanz, insbesondere Weißgrad und brillantem Glanz, aufweisen wie die weit verbreiteten Polyethylenglykolstearate. Zudem sollen die Perlglanzwachse auch mit den anderen, sensiblen bzw. kritischen Inhaltsstoffen wie Siliconen eine ausreichende Verträglichkeit aufweisen, so dass die Stabilität der Reinigungsmittel nicht beeinträchtigt wird. Des Weiteren wird erwartet, dass man die Perlglanzmittel leicht, d.h. ohne großen technischen und energetischen Aufwand in die kosmetische Formulierung einarbeiten kann. Dies soll möglichst einfach durch Rühren bei Raumtemperatur, ohne zeit- und kostenintensive Erwärmung der kosmetischen Formulierung erfolgen. Zusätzlich besteht bei vielen Herstellern der kosmetischen Mittel ein Bedürfnis nach Perlglanzwachsen, die als Konzentrat formulierbar sind, um unnötige Transport- und Lagerkosten zu vermeiden. Diese Konzentrate sollen zusätzlich fließfähig sein, um eine leichte Herstellbarkeit der kosmetischen Formulierung zu gewährleisten.

Gewünscht werden im Zuge der Nachhaltigkeit zudem Verbindungen, die ökologisch nachhaltig und ausreichend gut biologisch abbaubar sind.

Erfindungsgemäß wurden nun spezielle Zusammensetzungen, die Derivate des Isosorbids enthalten, entwickelt, die in Kombination mit nichtionischen Tensiden vorteilhafterweise in kosmetischen Mitteln oder Waschmitteln eingesetzt werden können.

Demgemäß betrifft die vorliegende Erfindung eine Zusammensetzung, umfassend mindestens einen Perlglanzwachs als ersten Bestandteil, enthaltend mindestens einen Isosorbiddiester einer gesättigten C₁₂-C₂₄ Fettsäure, und mindestens einen zweiten Bestandteil, enthaltend ein nichtionisches Tensid.

Erfindungsgemäß wurde herausgefunden, dass eine Zusammensetzung, die einen Perlglanzwachs mit mindestens einem Isosorbiddiester einer gesättigten C₁₂-C₂₄ Fettsäure und mindestens ein nichtionisches Tensid enthalten, einen verbesserten Perlglanzeffekt in kosmetischen Zusammensetzungen bewirkt.

Die vorliegende Erfindung wird nunmehr im Detail beschrieben.

### Perlglanzwachs (erster Bestandteil der erfindungsgemäßen Zusammensetzung)

Die erfindungsgemäße Zusammensetzung enthält als ersten Bestandteil ein Perlglanzwachs. Im Sinne der Erfindung wird der Begriff "Perlglanzwachs" für wachsartige Verbindungen oder Mischungen von Verbindungen verwendet, die einen Perlglanzeffekt bewirken (anstelle des Begriffes "Perlglanz" sind auch die Begriffe "Perlmuttglanz" oder "Lüster" gebräuchlich). Der Perlglanzeffekt wird im Sinne der Erfindung als Weißgrad und Brillanz, die Intensität des Schimmers, optisch im Vergleich zu dem Standard Ethylenglykoldistearat (EGDS) als Perlglanzwachs beurteilt.

### Isosorbiddiester als Bestandteil des Perlglanzwachses

Das Perlglanzwachs als erster Bestandteil der erfindungsgemäßen Zusammensetzung enthält zumindest einen Isosorbiddiester einer gesättigten C₁₂-C₂₄ Fettsäure. Isosorbid (oder 1,4;3,6-Dianhydrosorbitol) ist das Anhydrid des Sorbitols und kommerziell erhältlich. Es kann beispielsweise durch Erwärmen von Sorbitol in Gegenwart von konzentrierter Schwefel- oder Salzsäure erhalten werden. Darüber hinaus kann Isosorbid ausgehend von geeigneten Polysacchariden nach Hydrolyse zu D-Glucose und anschließender Reduktion zu D-Sorbitol durch intramolekulare, zweifache Dehydratisierung erhalten werden. Als Rohstoffquelle wird großtechnisch Stärke oder Cellulose verwendet. Isosorbid ist für Anwendungen im kosmetischen Bereich und im Waschmittelbereich ein interessanter Baustein, da er aus nachwachsenden Rohstoffen hergestellt wird.

Durch an sich dem Fachmann bekannte Verfahren können verscheiden Mono- und/oder Diester des Isosorbids erhalten werden.

Die erfindungsgemäß in dem Perlglanzwachs zu verwendenden Isosorbiddiester weisen die allgemeine Formel (I) auf 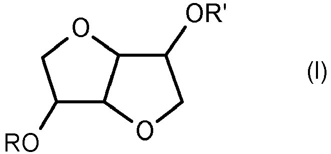 wobei R und R', unabhängig voneinander, jeweils für einen Rest COR" stehen, wobei R" für einen linearen gesättigten Alkylrest mit 11 bis 23 Kohlenstoffatomen steht. Bei dem erfindungsgemäß zu verwendenden Isosorbiddiester kann es sich um einen homogenen oder gemischten Diester des Isosorbids handeln.

Die oben dargestellte allgemeine Formel (I) umfasst im Rahmen der vorliegenden Erfindung auch alle Stereoisomere des Isosorbids, insbesondere Isoidid und Isomannid, sowie beliebige Mischungen davon. Ferner umfasst die allgemeinen Formel (I) auch alle Kombinationen der Reste R und R' untereinander. Daher wird im Rahmen der vorliegenden Erfindung unter dem Begriff "eines Isosorbiddiesters einer gesättigten Fettsäure" auch ein Diester des Isosorbids verstanden, der mit zwei unterschiedlichen gesättigten Fettsäuren verestert wurde.

Das Perlglanzwachs umfasst einen Isosorbiddiester einer gesättigten C₁₂ bis C₂₄-Fettsäure der allgemeinen Formel (I), welche bereits vorstehend beschrieben wurde.

Als Fettsäuren kommen zur Bildung des Isosorbiddiesters die gesättigten C₁₂- bis C₂₄-Fettsäuren in Frage. Diese werden vorzugsweise ausgewählt aus der Gruppe bestehend aus Laurinsäure, Tridecansäure, Myristinsäure, Pentadecansäure, Palmitinsäure, Margarinsäure, Stearinsäure, Nonadecansäure, Arachinsäure, Heneicosansäure, Behensäure und Lignocerinsäure.

In einer bevorzugten Ausgestaltung handelt es sich bei dem Isosorbiddiester um einen Diester von Isosorbid mit einer gesättigten C₁₂- bis C₂₂-Fettsäure, weiter bevorzugt um einen Diester von Isosorbid mit einer gesättigten C₁₂- bis C₂₀-Fettsäure, noch weiter bevorzugt um einen Diester von Isosorbid mit einer gesättigten C₁₆- bis C₁₈-Fettsäure.

Im Rahmen der vorliegenden Erfindung haben sich insbesondere die Diester von Isosorbid mit einer C₁₆- bis C₁₈-Fettsäure als geeignet erwiesen, die gewünschten Perlglanzeigenschaften in kosmetischen Zusammensetzungen zu erreichen.

Daher umfasst in einer weiteren noch bevorzugteren Ausgestaltung der erfindungsgemäßen Zusammensetzung der Perlglanzwachs einen Isosorbiddiester, der ausgewählt wird aus der Gruppe, bestehend aus Isosorbiddistearat, Isosorbiddipalmitat, Isosorbidpalmitatstearat und Mischungen der vorgenannten Verbindungen.

Des Weiteren umfasst das Perlglanzwachs der erfindungsgemäßen Zusammensetzung insbesondere eine Mischung von Diestern des Isosorbids, wobei eine Mischung von Isosorbiddiestern, die Isosorbiddistearat enthält, besonders bevorzugt ist.

In einer ganz besonders bevorzugten Ausgestaltung der erfindungsgemäßen Zusammensetzung umfasst das Perlglanzwachs eine Mischung von Isosorbiddistearat, Isosorbiddipalmitat und Isosorbidpalmitatstearat.

Wird im Rahmen der vorliegenden Erfindung als Perlglanzwachs diese besonders bevorzugte Mischung aus Isosorbiddistearat, Isosorbiddipalmitat und Isosorbidpalmitatstearat verwendet, so beträgt das Gewichtsverhältnis von Isosorbiddipalmitat zu Isosorbiddistearat vorzugsweise 45 : 55 bis 1 : 99, weiter bevorzugt 40 : 60 bis 1 : 99, noch weiter bevorzugt 30 : 70 bis 1 : 99, noch weiter bevorzugt 30 : 70 bis 2 : 98. Bei der Verwendung einer Mischung von Isosorbiddistearat und Isosorbiddipalmitat mit den vorstehend genannten relativen Verhältnissen, d.h. insbesondere mit einem Überschuss an Isosorbiddistearat, sind die Perlglanzeigenschaften ganz besonders ausgeprägt.

Der Isosorbiddiester kann in der erfindungsgemäßen Zusammensetzung in einer Menge von mindestens 70 Gew.-%, weiter bevorzugt mindestens 75 Gew.-%, noch weiter bevorzugt mindestens 80 Gew.-%, noch weiter bevorzugt mindestens 82 Gew.-%, jeweils bezogen auf den Perlglanzwachs als ersten Bestandteil der erfindungsgemäßen Zusammensetzung, enthalten sein.

Der Isosorbiddiester kann in der erfindungsgemäßen Zusammensetzung in einer Menge von höchstens 95 Gew.-%, weiter bevorzugt höchstens 90 Gew.-%, noch weiter bevorzugt höchstens 88 Gew.-%, jeweils bezogen auf den Perlglanzwachs als ersten Bestandteil der erfindungsgemäßen Zusammensetzung, enthalten sein.

Bevorzugt ist der Isosorbiddiester in einer Menge von 75 Gew.-% bis 95 Gew.-%, weiter bevorzugt 80 Gew.-% bis 90 Gew.-%, noch weiter bevorzugt 82 Gew.-% bis 88 Gew.-%, jeweils bezogen auf den Perlglanzwachs als ersten Bestandteil der erfindungsgemäßen Zusammensetzung, in der erfindungsgemäßen Zusammensetzung enthalten, um einen guten Perlglanzeffekt zu erreichen.

### Isosorbidmonoester als Bestandteil des Perlglanzwachses

In dem erfindungsgemäß zu verwendenden Perlglanzwachs kann ferner ein Isosorbidmonoester enthalten sein. Hierbei handelt es sich vorzugsweise um einen Monoester von Isosorbid mit einer gesättigten C₁₂- bis C₂₄-Fettsäure der allgemeinen Formel (II): 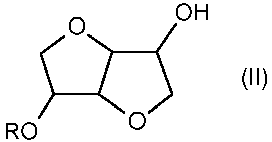 wobei R für einen Rest COR' steht, wobei R' für einen linearen gesättigten Alkylrest mit 11 bis 23 C-Atomen steht.

Für die Herstellung des Isosorbidmonoesters kommen die gleichen Fettsäuren in Frage, die auch vorstehend für den Isosorbiddiester verwendet werden. Insoweit wird auf obige Ausführungen verwiesen.

Wenn in dem Perlglanzwachs ein Isosorbidmonoester enthalten ist, so handelt es sich vorzugsweise um einen Isosorbidmonoester mit einer C₁₆- bis C₁₈-Fettsäure.

Insbesondere kann es sich um einen Isosorbidmonoester handeln, der ausgewählt wird aus der Gruppe, bestehend aus Isosorbidmonostearat, Isosorbidmonopalmitat und Mischungen der vorgenannten Verbindungen.

Des Weiteren kann das Perlglanzwachs der erfindungsgemäßen Zusammensetzung insbesondere eine Mischung von Monoestern des Isosorbids enthalten, wobei eine Mischung von Isosorbidmonoestern, die Isosorbidmonostearat enthält, besonders bevorzugt ist.

In einer ganz besonders bevorzugten Ausgestaltung handelt es sich bei dem Isosorbidmonoester des Perlglanzwachses um eine Mischung von Isosorbidmonostearat und Isosorbidmonopalmitat.

Wird im Rahmen der vorliegenden Erfindung im Perlglanzwachs diese Mischung aus Isosorbidmonostearat und Isosorbidmonopalmitat verwendet, so beträgt das Gewichtsverhältnis von Isosorbidmonopalmitat zu Isosorbidmonostearat vorzugsweise 45 : 55 bis 1 : 99, weiter bevorzugt 40 : 60 bis 1 : 99, noch weiter bevorzugt 30 : 70 bis 1 : 99, noch weiter bevorzugt 30 : 70 bis 2 : 98. Bei der Verwendung einer Mischung von Isosorbidmonostearat und Isosorbidmonopalmitat in den vorstehend genannten relativen Verhältnissen sind die Perlglanzeigenschaften ganz besonders ausgeprägt.

Der Isosorbidmonoester kann in der erfindungsgemäßen Zusammensetzung in einer Menge von mindestens 0,01 Gew.-%, weiter bevorzugt mindestens 0,5 Gew.-%, noch weiter bevorzugt mindestens 1 Gew.-%, jeweils bezogen auf den Perlglanzwachs als ersten Bestandteil der erfindungsgemäßen Zusammensetzung, enthalten sein.

Der Isosorbidmonoester kann in der erfindungsgemäßen Zusammensetzung in einer Menge von höchstens 20 Gew.-%, weiter bevorzugt höchstens 15 Gew.-%, noch weiter bevorzugt höchstens 10 Gew.-%, jeweils bezogen auf den Perlglanzwachs als ersten Bestandteil der erfindungsgemäßen Zusammensetzung, enthalten sein.

Bevorzugt ist der Isosorbidmonoester in einer Menge von 0,01 Gew.-% bis 20 Gew.-%, weiter bevorzugt 0,5 Gew.-% bis 15 Gew.-%, noch weiter bevorzugt 1 Gew.-% bis 10 Gew.-%, jeweils bezogen auf den Perlglanzwachs als ersten Bestandteil der erfindungsgemäßen Zusammensetzung, in der erfindungsgemäßen Zusammensetzung enthalten, um einen guten Perlglanzeffekt zu erreichen.

### Fettsäure als Bestandteil des Perlglanzwachses

In dem Perlglanzwachs der erfindungsgemäßen Zusammensetzung kann des Weiteren eine Fettsäure enthalten sein. Hierbei handelt es sich vorzugsweise um eine Fettsäure, die vorstehend bereits zur Herstellung des Isosorbiddiesters bzw. des Isosorbidmonoesters verwendet wurde. Insoweit wird auf obige Ausführungen verwiesen.

Wenn das Perlglanzwachs eine Fettsäure enthält, so handelt es sich vorzugsweise um eine C₁₆- bis C₁₈-Fettsäure, welche besonders bevorzugt ausgewählt ist aus der Gruppe, bestehend aus Stearinsäure, Palmitinsäure und Mischungen davon.

In einer ganz besonders bevorzugten Ausgestaltung handelt es sich bei der Fettsäure und eine Mischung von Stearinsäure und Palmitinsäure.

Wird im Rahmen der vorliegenden Erfindung im Perlglanzwachs eine Mischung aus Stearinsäure und Palmitinsäure verwendet, so beträgt das Gewichtsverhältnis von Palmitinsäure zu Stearinsäure vorzugsweise 45 : 55 bis 1 : 99, weiter bevorzugt 40 : 60 bis 1 : 99, noch weiter bevorzugt 30 : 70 bis 1 : 99, noch weiter bevorzugt 30 : 70 bis 2 : 98.

Die Fettsäure kann in der erfindungsgemäßen Zusammensetzung in einer Menge von höchstens 20 Gew.-%, weiter bevorzugt höchstens 17 Gew.-%, noch weiter bevorzugt höchstens 14 Gew.-%, jeweils bezogen auf den Perlglanzwachs als ersten Bestandteil der erfindungsgemäßen Zusammensetzung, enthalten sein.

Die Fettsäure kann in der erfindungsgemäßen Zusammensetzung in einer Menge von mindestens 1 Gew.-%, weiter bevorzugt mindestens 3 Gew.-%, noch weiter bevorzugt mindestens 5 Gew.-%, jeweils bezogen auf den Perlglanzwachs als ersten Bestandteil der erfindungsgemäßen Zusammensetzung, enthalten sein.

Bevorzugt ist die Fettsäure in einer Menge von 1 Gew.-% bis 20 Gew.-%, weiter bevorzugt 3 Gew.-% bis 17 Gew.-%, noch weiter bevorzugt 5 Gew.-% bis 14 Gew.-%, jeweils bezogen auf den Perlglanzwachs als ersten Bestandteil der erfindungsgemäßen Zusammensetzung, in der erfindungsgemäßen Wachszusammensetzung des Perlglanzkonzentrates enthalten.

### Nichtionisches Tensid (zweiter Bestandteil der erfindungsgemäßen Zusammensetzung)

In der erfindungsgemäßen Zusammensetzung wird als zweiter Bestandteil ein nichtionisches Tensid verwendet.

Im Rahmen der vorliegenden Erfindung wird unter dem Begriff eines "nichtionischen Tensids" auch eine Mischung von zwei oder mehreren nichtionischen Tensiden verstanden.

Bei dem nichtionischen Tensid handelt es sich beispielsweise um Fettalkoholpolyglykolether; Alkylphenolpolyglykolether; Fettsäurepolyglykolester; Fettsäureamidpolyglykolether; Fettaminpolyglykolether; Polyolfettester, insbesondere Fettsäureglycerinester, speziell Fettsäureglycerinmonoester, Fettsäureglycerindiester und Fettsäureglycerintriester, wobei der Gehalt an Fettsäuremonoester in solch einer Mischung mehr als 40 Gew.-% beträgt; alkoxylierte Triglyceride; Mischether bzw. Mischformale; gegebenenfalls partiell oxidierte Alk(en)ylpolyglykoside bzw. Glucoronsäurederivate; Fettsäure-N-alkylglucamide; Proteinhydrolysate (insbesondere pflanzliche Produkte auf Weizenbasis); Polyolfettsäureester; Zuckerester; Sorbitanester; Polysorbate und Aminoxide.

Sofern die nichtionischen Tenside Polyglycoletherketten enthalten, können diese eine konventionelle, vorzugsweise jedoch eine eingeengte Homologenverteilung aufweisen.

Bei dem nichtionischen Tensid handelt es sich bevorzugt um ein von Ethylenoxideinheiten freies nichtionisches Tensid.

Bevorzugt sind des Weiterem Fettsäureglycerinester, speziell Fettsäureglycerinmonoester, Fettsäureglycerindiester und Fettsäureglycerintriester, wobei der Gehalt an Monoester in der Mischung aus Mono-, Di- und Triester mehr als 40 Gew.-% beträgt, und Alkylpolyglykoside.

In einer ganz besonders bevorzugten Ausführungsform handelt es sich bei dem nichtionischen Tensid um ein Alkylpolyglykosid.

Alkylpolyglykoside stellen bekannte nichtionische Tenside dar, die der Formel (III) folgen,

R¹O-[G]ₚ (III)

in der
- R¹ für einen Alkylrest mit 4 bis 22 Kohlenstoffatomen,
- G für einen Zuckerrest mit 5 oder 6 Kohlenstoffatomen und
- p für Zahlen von 1 bis 10 steht.

Sie können nach den einschlägigen Verfahren der präparativen organischen Chemie erhalten werden. Stellvertretend für das umfangreiche Schrifttum sei hier auf die Übersichtsarbeit von Biermann et al. in Starch/Stärke 45, 281 (1993**),** B.Salka in Cosm.Toil. 108, 89 (1993**)** sowie J.Kahre et al. in SÖFW-Journal Heft 8, 598 (1995**)** verwiesen.

Die Alkylpolyglykoside können sich von Aldosen bzw. Ketosen mit 5 oder 6 Kohlenstoffatomen, vorzugsweise der Glucose, ableiten. Die bevorzugten Alkylpolyglykoside sind somit Alkylpolygoglucoside. Die Indexzahl p in der allgemeinen Formel (I) gibt den Polymerisierungsgrad (DP), d.h. die Verteilung von Mono- und Polygoglykosiden an, und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkylpolyglykosid eine analytisch ermittelte rechnerische Größe, die meistens eine gebrochene Zahl darstellt. Vorzugsweise werden Alkylpolyglykoside mit einem mittleren Polymerisierungsgrad p von 1,1 bis 3,0 eingesetzt. Aus anwendungstechnischer Sicht sind solche Alkylpolyglykoside bevorzugt, deren Polymerisierungsgrad kleiner als 1,7 ist und insbesondere zwischen 1,2 und 1,7 liegt.

Der Alkylrest R¹ kann sich von primären Alkoholen mit 4 bis 22, vorzugsweise 6 bis 18 Kohlenstoffatomen ableiten.

In einer Ausführungsform der vorliegenden Erfindung leiten sich der Alkylrest R¹ von niederen primären Alkoholen mit vorzugsweise 4 bis 11, weiter bevorzugt 8 oder 10 Kohlenstoffatomen ab.

Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkylpolyglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkylpolyglucoside auf Basis technischer C₉/₁₁-Oxoalkohole (DP = 1 bis 3).

Der Alkylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinyalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkylpolyglucoside auf Basis von gehärtetem C₁₂/₁₄-Kokosalkohol mit einem DP von 1 bis 3.

Ganz besonders bevorzugt im Sinne der vorliegenden Erfindung sind Zusammensetzungen, enthaltend eine Mischung von verschiedenen Alkylpolyglykosiden der Formel (III), in welcher sich R¹ eines Alkylpolyglykosids von einem niederen primären Alkohol mit 4 bis 11 Kohlenstoffatomen, vorzugsweise 8 und/oder 10 Kohlenstoffatomen, und in welcher sich R¹ des anderen Alkylpolyglykosids von einem primären höheren Alkohol mit 12 bis 22 Kohlenstoffatomen, vorzugsweise 12 bis 16 Kohlenstoffatomen ableiten.

Im Rahmen der vorliegenden Erfindung sind als Bestandteil II insbesondere Mischungen von verschiedenen Alkylpolyglykosiden der Formel (III) bevorzugt, in der sich R¹ von primären Alkoholmischungen ableiten, die zu 10 bis 50 Gew.-% 8 und 10 Kohlenstoffatome und zu 50 bis 90 Gew.-% 12 bis 16 Kohlenstoffatome aufweisen, wobei in geringen Mengen, insbesondere unter 10 Gew.-%, bezogen auf die Alkoholmischung, sie sich von Alkoholen mit weniger oder mehr Kohlenstoffatomen ableiten lassen.

Derartige Mischungen der verschiedenen Alkylpolyglykosiden können durch Abmischen der Alkylpolyglykoside oder durch Einsatz derartiger Alkoholmischungen bei der Herstellung der Alkylpolyglykoside erhalten werden.

### Bevorzugte Ausführungsformen der erfindungsgemäßen Zusammensetzung

In Folgenden werden besonders bevorzugte Ausführungsformen der erfindungsgemäßen Zusammensetzung beschrieben.

Das Perlglanzwachs kann als erster Bestandteil der erfindungsgemäßen Zusammensetzung in einer Menge von mindestens 10 Gew.-%, weiter bevorzugt mindestens 15 Gew.-%, noch weiter bevorzugt mindestens 18 Gew.-%, jeweils bezogen auf die Zusammensetzung, enthalten sein.

Das Perlglanzwachs kann in der erfindungsgemäßen Zusammensetzung in einer Menge von höchstens 40 Gew.-%, weiter bevorzugt höchstens 35 Gew.-%, noch weiter bevorzugt höchstens 30 Gew.-%, jeweils bezogen auf die Zusammensetzung, enthalten sein.

Bevorzugt ist der Perlglanzwachs in einer Menge von 10 Gew.-% bis 40 Gew.-%, weiter bevorzugt 15 Gew.-% bis 35 Gew.-%, noch weiter bevorzugt 18 Gew.-% bis 35 Gew.-%, jeweils bezogen auf die Zusammensetzung, in der erfindungsgemäßen Zusammensetzung enthalten.

Das nichtionische Tensid kann als zweiter Bestandteil der erfindungsgemäßen Zusammensetzung in einer Menge von mindestens 5 Gew.-%, weiter bevorzugt mindestens 10 Gew.-%, noch weiter bevorzugt mindestens 12 Gew.-%, jeweils bezogen auf die Zusammensetzung, enthalten sein.

Das nichtionische Tensid kann in der erfindungsgemäßen Zusammensetzung in einer Menge von höchstens 40 Gew.-%, weiter bevorzugt höchstens 35 Gew.-%, noch weiter bevorzugt höchstens 30 Gew.-%, jeweils bezogen auf die Zusammensetzung, enthalten sein.

Bevorzugt ist das nichtionische Tensid in einer Menge von 5 Gew.-% bis 40 Gew.-%, weiter bevorzugt 10 Gew.-% bis 35 Gew.-%, noch weiter bevorzugt 12 Gew.-% bis 30 Gew.-%, jeweils bezogen auf die Zusammensetzung, in der erfindungsgemäßen Zusammensetzung enthalten.

In der erfindungsgemäßen Zusammensetzung beträgt
- das Gewichtsverhältnis von Isosorbiddipalmitat zu Isosorbiddistearat vorzugsweise 45 : 55 bis 1 : 99 und/oder
- das Gewichtsverhältnis von Isosorbidmonopalmitat zu Isosorbidmonostearat vorzugsweise 45 : 55 bis 1 : 99 und/oder
- das Gewichtsverhältnis von Palmitinsäure zu Stearinsäure vorzugsweise 45 : 55 bis 1 : 99 beträgt.

In einer ersten besonders bevorzugten Ausführungsform enthält die erfindungsgemäße Zusammensetzung daher
- mindestens einen wie zuvor beschriebenen Perlglanzwachs in einer Menge von 10 bis 40 Gew.-%; und
- mindestens ein nichtionisches Tensid in einer Menge von 5 bis 40 Gew.-%,
wobei die Mengenangaben jeweils auf die Zusammensetzung bezogen sind.

In einer zweiten besonders bevorzugten Ausführungsform enthält die erfindungsgemäße Zusammensetzung
- mindestens einen wie zuvor beschriebenen Perlglanzwachs in einer Menge von 15 bis 35 Gew.-%; und
- mindestens ein nichtionisches Tensid in einer Menge von 10 bis 35 Gew.-%,
wobei die Mengenangaben jeweils auf die Zusammensetzung bezogen sind.

In einer dritten bevorzugten Ausführungsform enthält die erfindungsgemäße Zusammensetzung
- mindestens einen wie zuvor beschriebenen Perlglanzwachs in einer Menge von 18 bis 30 Gew.-%; und
- mindestens ein nichtionisches Tensid in einer Menge von 12 bis 30 Gew.-%,
wobei die Mengenangaben jeweils auf die Zusammensetzung bezogen sind.

In den zuvor beschriebenen ersten bis dritten Ausführungsformen sind die erfindungsgemäßen Zusammensetzungen insbesondere dadurch gekennzeichnet, dass der Perlglanzwachs eine Mischung aus Isosorbiddistearat, Isosorbiddipalmitat und Isosorbidpalmitatstearat als Isosorbiddiester, eine Mischung aus Isosorbidmonostearat und Isosorbidmonopalmitat als Isosorbidmonoester und um eine Mischung aus Stearinsäure und Palmitinsäure als Fettsäure enthält und der das mindestens eine nichtionische Tensid ein Alkylpolyglykosid umfasst.

In den zuvor beschriebenen ersten bis dritten Ausführungsformen sind die erfindungsgemäßen Zusammensetzungen insbesondere auch dadurch gekennzeichnet, dass in dem der erfindungsgemäßen Zusammensetzung der Isosorbiddiester in einer Menge von mindestens 70 Gew.-%, bezogen auf den Perlglanzwachs, enthaltend ist.

In den zuvor beschriebenen ersten bis dritten Ausführungsformen sind die erfindungsgemäßen Zusammensetzungen insbesondere auch dadurch gekennzeichnet,
- dass der Perlglanzwachs eine Mischung aus Isosorbiddistearat, Isosorbiddipalmitat und Isosorbidpalmitatstearat als Isosorbiddiester, eine Mischung aus Isosorbidmonostearat und Isosorbidmonopalmitat als Isosorbidmonoester und um eine Mischung aus Stearinsäure und Palmitinsäure als Fettsäure enthält;
- dass mindestens ein nichtionisches Tensid ein Alkylpolyglykosid umfasst;
- dass der Isosorbiddiester in einer Menge von 75 Gew.-% bis 95 Gew.-%, weiter bevorzugt 80 Gew.-% bis 90 Gew.-%, noch weiter bevorzugt 82 Gew.-% bis 88 Gew.-%, jeweils bezogen auf den Perlglanzwachs als ersten Bestandteil der erfindungsgemäßen Zusammensetzung, in der erfindungsgemäßen Zusammensetzung enthalten ist.

In den zuvor beschriebenen ersten bis dritten Ausführungsformen sind die erfindungsgemäßen Zusammensetzungen insbesondere auch dadurch gekennzeichnet,
- dass der Perlglanzwachs eine Mischung aus Isosorbiddistearat, Isosorbiddipalmitat und Isosorbidpalmitatstearat als Isosorbiddiester, eine Mischung aus Isosorbidmonostearat und Isosorbidmonopalmitat als Isosorbidmonoester und um eine Mischung aus Stearinsäure und Palmitinsäure als Fettsäure enthält;
- dass mindestens eine nichtionische Tensid ein Alkylpolyglykosid umfasst;
- dass das Verhältnis von Isosorbiddipalmitat zu Isosorbiddistearat 45 : 55 bis 1 : 99, weiter bevorzugt 40 : 60 bis 1 : 99, noch weiter bevorzugt 30 : 70 bis 1 : 99, noch weiter bevorzugt 30 : 70 bis 2 : 98, beträgt;
- dass das Verhältnis Isosorbidmonopalmitat zu Isosorbidmonostearat 45 : 55 bis 1 : 99, weiter bevorzugt 40 : 60 bis 1 : 99, noch weiter bevorzugt 30 : 70 bis 1 : 99, noch weiter bevorzugt 30 : 70 bis 2 : 98, beträgt; und
- dass das Verhältnis Palmitinsäure zu Stearinsäure 45 : 55 bis 1 : 99, weiter bevorzugt 40 : 60 bis 1:99, noch weiter bevorzugt 30 : 70 bis 1 : 99, noch weiter bevorzugt 30 : 70 bis 2 : 98, beträgt.

In den zuvor beschriebenen ersten bis dritten Ausführungsformen sind die erfindungsgemäßen Zusammensetzungen darüber hinaus insbesondere dadurch gekennzeichnet, dass der Gehalt an Fettsäure in der Zusammensetzung höchstens 10 Gew.-%, noch weiter bevorzugt höchstens 5 Gew.-%, jeweils bezogen auf die erfindungsgemäße Zusammensetzung, beträgt.

In einer ganz besonders bevorzugten Ausführungsform der vorliegenden Erfindung umfasst die erfindungsgemäße Zusammensetzung
10 bis 40 Gew.-% eines Perlglanzwachses als ersten Bestandteil, enthaltend
- Isosorbiddistearat, Isosorbiddipalmitat und Isosorbidpalmitatstearat in einer Menge von 75 bis 95 Gew.-%, bezogen auf den Perlglanzwachs, mit einem Verhältnis von Isosorbiddipalmitat zu Isosorbiddistearat von 30 : 70 bis 2 : 98;
- Isosorbidmonostearat und Isosorbidmonopalmitat in einer Menge von 0,01 bis 20 Gew.-%, bezogen auf den Perlglanzwachs, mit einem Verhältnis von Isosorbidmonopalmitat zu Isosorbidmonostearat von 30 : 70 bis 2 : 98;
- Stearinsäure und Palmitinsäure in einer Menge von 1 bis 30 Gew.-%, bezogen auf den Perlglanzwachs, mit einem Verhältnis von Palmitinsäure zu Stearinsäure von 30 : 70 bis 2 : 98; und
5 bis 40 Gew.-% eines Polyalkylglykosid als nichtionisches Tensid als zweiten Bestandteil,
wobei die Mengenangaben an erstem und zweitem Bestandteil auf die Zusammensetzung bezogen sind.

In einer weiteren ganz besonders bevorzugten Ausführungsform der vorliegenden Erfindung umfasst die erfindungsgemäße Zusammensetzung
15 bis 35 Gew.-% eines Perlglanzwachses als ersten Bestandteil, enthaltend
- Isosorbiddistearat, Isosorbiddipalmitat und Isosorbidpalmitatstearat in einer Menge von 80 bis 90 Gew.-%, bezogen auf den Perlglanzwachs, mit einem Verhältnis von Isosorbiddipalmitat zu Isosorbiddistearat von 30 : 70 bis 2 : 98;
- Isosorbidmonostearat und Isosorbidmonopalmitat in einer Menge von 0,5 bis 15 Gew.-%, bezogen auf den Perlglanzwachs, mit einem Verhältnis von Isosorbidmonopalmitat zu Isosorbidmonostearat von 30 : 70 bis 2 : 98;
- Stearinsäure und Palmitinsäure in einer Menge von 3 bis 25 Gew.-%, bezogen auf den Perlglanzwachs, mit einem Verhältnis von Palmitinsäure zu Stearinsäure von 30 : 70 bis 2 : 98; und
10 bis 35 Gew.-% eines Polyalkylglykosid als nichtionisches Tensid als zweiten Bestandteil,
wobei die Mengenangaben an erstem und zweitem Bestandteil auf die Zusammensetzung bezogen sind.

In einer ganz besonders bevorzugten Ausführungsform der vorliegenden Erfindung umfasst die erfindungsgemäße Zusammensetzung
18 bis 30 Gew.-% eines Perlglanzwachses als ersten Bestandteil, enthaltend
- Isosorbiddistearat, Isosorbiddipalmitat und Isosorbidpalmitatstearat in einer Menge von 82 bis 88 Gew.-%, bezogen auf den Perlglanzwachs, mit einem Verhältnis von Isosorbiddipalmitat zu Isosorbiddistearat von 30 : 70 bis 2 : 98;
- Isosorbidmonostearat und Isosorbidmonopalmitat in einer Menge von 1 bis 10 Gew.-%, bezogen auf den Perlglanzwachs, mit einem Verhältnis von Isosorbidmonopalmitat zu Isosorbidmonostearat von 30 : 70 bis 2 : 98;
- Stearinsäure und Palmitinsäure in einer Menge von 5 bis 20 Gew.-%, bezogen auf den Perlglanzwachs, mit einem Verhältnis von Palmitinsäure zu Stearinsäure von 30 : 70 bis 2 : 98; und
12 bis 30 Gew.-% eines Polyalkylglykosid als nichtionisches Tensid als zweiten Bestandteil,
wobei die Mengenangaben an erstem und zweitem Bestandteil auf die Zusammensetzung bezogen sind.

In einer weiteren bevorzugten unabhängigen Ausführungsform der vorliegenden Erfindung ist die erfindungsgemäße Zusammensetzung dadurch gekennzeichnet, dass das Gewichtsverhältnis von Diestern des Isosorbids zu Monoestern des Isosorbids in der Zusammensetzung mindestens 4 : 1, weiter bevorzugt mindestens 6 : 1, noch weiter bevorzugt mindestens 8 : 1, noch weiter bevorzugt mindestens 10 : 1, beträgt.

Die erfindungsgemäße Zusammensetzung wird insbesondere als Perlglanzkonzentrat verwendet, in welcher sie mit Polyolen und Wasser formuliert wird.

Die diesen Perlglanzkonzentraten können die Polyole in Mengen von bis 40 Gew.-%, insbesondere 0,1 bis 30 Gew.-%, weiter bevorzugt 0,2 bis 20 Gew.-%, jeweils bezogen auf das Perlglanzkonzentrat, vorliegen.

Polyole, die hier in Betracht kommen, besitzen vorzugsweise 2 bis 15 Kohlenstoffatome und mindestens zwei Hydroxylgruppen. Insbesondere geeignete Polyole sind Glycerin und/oder Sorbitol.

Des Weiteren ist noch Wasser ad 100 Gew.-% enthalten.

Daher resultiert erfindungsgemäß insbesondere ein Perlglanzkonzentrat, welches
(a) 10 bis 40 Gew.-% Perlglanzwachs;
(b) 5 bis 40 Gew.-% nichtionisches Tensid;
(c) 0 bis 40 Gew.-% Polyol; und
(d) ad 100 Gew.-% Wasser
enthält.

Im Rahmen der vorliegenden Erfindung wird unter einem Perlglanzkonzentrat eine Mischung der erfindungsgemäßen Zusammensetzung in Wasser mit gegebenenfalls zusätzlich verwendetem Polyol verstanden. Dieses Perlglanzkonzentrat kann in wässrige, tensidische Formulierungen eingebracht werden, welche dann handelsübliche Formulierungen darstellen, die zur finalen Anwendung als kosmetische Mittel dienen.

Die gegebenenfalls mit Polyol und Wasser formulierten Perlglanzkonzentrate können erfindungsgemäß hergestellt werden, indem man
- den Perlglanzwachs als ersten Bestandteil sowie die nichtionischen Tenside als zweiten Bestandteil sowie ggf. die Polyole und ad 100 Gew. % Wasser unter Rühren auf Temperaturen erwärmt, die 5 bis 20 °C über dem Schmelzpunkt des Perlglanzwachs liegen,
- die Mischung bei diesen Temperaturen rührt und
- anschließend die Mischung unter stetigem Rühren auf etwa Raumtemperatur (20 bis 23 °C) abkühlt.

Es ist auch möglich die Perlglanzkonzentrate herzustellen, in dem man
- zu einer wässrigen Paste des nichtionischen Tensids den Perlglanzwach einrührt und auf Temperaturen, die 5 bis 20 °C über dem Schmelzpunkt des Perlglanzwachs liegen, erwärmt,
- die Mischung bei diesen Temperaturen rührt und
- anschließend mit weiterem Wasser und ggf. Polyolen die gewünschte Konzentration einstellt.

In der Regel empfiehlt sich eine Rührzeit der Mischung von etwa 15 bis 60 Minuten, bevor vorzugsweise die Mischung mit einer Kühlrate von etwa 10 bis 30 °C, vorzugsweise 15 bis 25 °C pro Stunde abgekühlt wird.

Die erfindungsgemäßen Perlglanzkonzentrate sind fließfähige Mischungen, die leicht in wässrige, tensidische Zubereitungen eingerührt werden können und dort einen brillanten Glanz mit hohem Weißgrad erzeugen.

Die in der erfindungsgemäßen Zusammensetzung verwendeten Isosorbidester können durch an sich bekannte Veresterungverfahren synthetisiert werden. In der WO 01/83488 A wird exemplarisch eine geeignete Methode offenbart mit welcher Mono- oder Diester des Isosorbids oder Mischungen aus Mono- und Diestern des Isosorbids erhalten werden können.

Die vorliegende Erfindung betrifft daher auch ein Verfahren zur Herstellung einer erfindungsgemäßen Zusammensetzung, das durch den Verfahrensschritt des Veresterns von Isosorbid mit mindestens einer gesättigten C₁₂-C₂₄ Fettsäure unter Erhalt eines Veresterungsprodukts gekennzeichnet ist.

Das erfindungsgemäße Verfahren kann dabei in Gegenwart eines Veresterungskatalysators durchgeführt werden, wobei Zinnoxalat einen geeigneten Katalysator darstellt.

Wenn im Rahmen der vorliegenden Erfindung ein Veresterungskatalysator verwendet wird, wird der verwendeten Veresterungskatalysator üblicherweise nach der Veresterungsreaktion deaktiviert, insbesondere wird der verwendete Veresterungskatalysator hydrolysiert.

Im Anschluss an die Veresterungsreaktion und der gegebenenfalls durchzuführenden Deaktivierung des Katalysators wird das resultierende Reaktionsprodukt üblicherweise aufgereinigt, beispielsweise durch Filtration oder Destillation unter Vakuum.

Die Veresterung selbst wird üblicherweise bei einer Temperatur von 160 bis 230 °C, weiter bevorzugt 170 bis 220 °C, noch weiter bevorzugt 180 bis 220 °C, durchgeführt.

Bei der Herstellung der Mono- und Diester des Isosorbids ist zu beachten, dass je nach Überschuss des eingesetzten Isosorbids bzw. der C₁₂-C₂₄ Fettsäure ein unterschiedliches Verhältnis an Mono- und Diester entsteht, da die beiden Hydroxylgruppen durch ihre exo- bzw. endo-Anordnung unterschiedlich reaktiv sind

In dem erfindungsgemäßen Verfahren wird üblicherweise ein Überschuss an C₁₂-C₂₄ Fettsäure zu Isosorbid von mindestens 2,05 Äquivalente Fettsäure bezogen auf 1 Äquivalent Isosorbid verwendet. Besonders bevorzugt ist ein Überschuss an Fettsäure zu Isosorbid von 2,05 bis 2,5 Äquivalente, weiter bevorzugt 2,1 bis 2,4 Äquivalente, noch weiter bevorzugt 2,1 bis 2,2 Äquivalente, jeweils bezogen auf 1 Äquivalent Isosorbid.

Das erfindungsgemäße Verfahren wird üblicherweise mit diesem Überschuss an C₁₂-C₂₄ Fettsäure so lange durchgeführt, bis die erfindungsgemäß definierten Mengen an Bestandteil (A), (B) und (C) in der beanspruchten Zusammensetzung erreicht werden. Dieses ist durch den Fachmann mit üblichen Maßnahmen, beispielsweise mittels GC-Kontrolle und Säurezahlbestimmung, feststellbar. Daher wird die erfindungsgemäße Zusammensetzung vorzugsweise in einer EintopfReaktion hergestellt, bei welcher der Isosorbiddiester und der Isosorbidmonoester ausgehend von Isosorbid und einer oder mehrerer C₁₂-C₂₄ Fettsäuren gleichzeitig gebildet werden. Durch Verwendung eines Überschusses an Fettsäure verbleibt von dieser ebenfalls ein Rest in der erfindungsgemäßen Zusammensetzung. Allerdings ist auch eine Herstellung der erfindungsgemäßen Zusammensetzung durch Mischung der Einzelbestandteile möglich.

### Verwendung in kosmetischen Mitteln

Die erfindungsgemäße Zusammensetzung kann bevorzugt als Perlglanzmittel in kosmetischen Mitteln, insbesondere tensidischen kosmetischen Mitteln, verwendet werden. Bei den kosmetischen Mitteln, insbesondere tensidischen kosmetischen Mitteln, handelt es sich im Allgemeinen um flüssige kosmetische Mittel.

Unter kosmetischen Mitteln sind hier alle dem Fachmann bekannten Mittel zu verstehen, die ausschließlich oder überwiegend dazu bestimmt sind, äußerlich am Körper des Menschen oder in seiner Mundhöhle zur Reinigung, Pflege, zum Schutz, zur Erhaltung eines guten Zustandes, zur Parfümierung, zur Veränderung des Aussehens oder dazu angewendet zu werden, den Körpergeruch zu beeinflussen.

Die erfindungsgemäßen kosmetischen Mittel können im Besonderen Formulierungen zur Körperpflege sein, z. B. eine Körpermilch, Cremes, Lotionen, sprühbare Emulsionen, Produkte zur Eliminierung des Körpergeruchs etc. Die Kohlenwasserstoffe lassen sich auch in tensidhaltigen Formulierungen wie z. B. Schaum- und Duschbädern, Haarshampoos und Pflegespülungen einsetzen. Je nach Applikationszweck enthalten die kosmetischen Formulierungen eine Reihe weiterer Hilfs- und Zusatzstoffe, wie beispielsweise Tenside, weitere Ölkörper, Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insekten-repellentien, Selbstbräuner, Tyrosinaseinhibitoren (Depigmentierungsmittel), Hydrotrope, Solubilisatoren, Konservierungsmittel, Parfümöle, Farbstoffe etc., die nachstehend exemplarisch aufgelistet sind.

Als weitere oberflächenaktive Stoffe (Tenside) können anionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten. Der Anteil der Tenside liegt hier üblicherweise bei etwa 1 bis 30, vorzugsweise 5 bis 25 und insbesondere 10 bis 20 Gew.-%.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

Körperpflegemittel, wie Cremes, Lotionen und Milchen, enthalten üblicherweise eine Reihe weiterer Ölkörper und Emollients, die dazu beitragen, die sensorischen Eigenschaften weiter zu optimieren. Die Ölkörper sind üblicherweise in einer Gesamtmenge von 1 bis 50 Gew.-%, vorzugsweise 5 bis 25 Gew.-% und insbesondere 5 bis 15 Gew.-% enthalten. Als weitere Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z. B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbe-henat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oley-lisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, E-rucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylyl Ether (Cetiol® OE), Ringöffnungsprodukte von epoxidierten Fettsäu-reestern mit Polyolen.

Fette und Wachse werden den Körperpflegeprodukten als Pflegestoffe zugesetzt und auch, um die Konsistenz der Kosmetika zu erhöhen. Typische Beispiele für Fette sind Glyceride, d. h. feste pflanzliche oder tierische Produkte, die im Wesentlichen aus gemischten Glycerinestern höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, - palmitat oder -stearat kommen hierfür in Frage. Als Wachse kommen u. a. natürliche Wachse, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z. B. Montanesterwachse, Sasolwachse, hydrierte Jojobawachse sowie synthetische Wachse, wie z. B. Polyalkylenwachse und Polyethylenglycolwachse in Frage. Neben den Fetten kommen als Zusatzstoffe auch fettähnliche Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als Verdickungsmittel eignen sich beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyvinylalkohol, Polyvinylpyrrolidon und Bentonite wie z. B. Bentone® Gel VS-5PC (Rheox).

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UV-B-Filter können öllöslich oder wasserlöslich sein. Als typische UV-A-Filter kommen insbesondere Derivate des Benzoylmethans in Frage. Die UV-A und UV-B-Filter können selbstverständlich auch in Mischungen eingesetzt werden, z.B. Kombinationen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) sowie Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Häufig werden derartige Kombinationen mit wasserlöslichen Filtern wie z. B. 2-Phenylbenzimidazol-5-sulfonsäure und deren Alkali-, Erdalkali-, Ammonium-, Alkylammonium-, Alkanolammonium- und Glucammoniumsalze kombiniert. Neben den genannten löslichen Stoffen kommen auch unlösliche Lichtschutzpigmente, nämlich feindisperse Metalloxide in Frage. Beispiele für geeignete Metalloxide sind insbesondere Zinkoxid und Titandioxid. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

Desodorierende Wirkstoffe wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker.

Als Insekten-Repellentien kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent® 3535 von der Merck KGaA vertrieben wird, sowie Butylacetylaminopropionate in Frage.

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als Parfümöle seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Die kosmetischen Mittel enthalten die erfindungsgemäß beanspruchten Zusammensetzungen als Perlglanzmittel. Allerdings können die kosmetischen Zusammensetzungen auch weitere Perlglanzmittel enthalten. In diesem Sinn kommen als Perlglanzwachse, insbesondere für den Einsatz in tensidischen Formulierungen, beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen (ohne die Sorbitanderivate) mit 2 bis 15 Kohlenstoffatomen und 2 bis 10 Hydroxylgruppen sowie deren Mischungen.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Stabilisatoren können Metallsalze von Fettsäuren, wie z. B. Magnesium-, Aluminium- und/ oder Zinkstearat bzw. -ricinoleat eingesetzt werden.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

Im Besonderen sind solche kosmetischen Mittel bevorzugt, die eine wässerige- und eine Ölphase nebeneinander aufweisen und z.B. in Form einer Emulsion (sowohl Wasser-in-ÖI, als auch Öl-in-Wasser) vorliegen und die als einen Bestandteil ein oder mehrere Isosorbid-Derivate gemäß der obigen Definition enthalten. Dabei können die Isosorbid-Derivate als Ölphase bzw. Emollient, oder als Bestandteil der Ölphase eingesetzt werden. Sie können aber, wie im Weiteren noch ausgeführt wird, in Abhängigkeit von ihrer Struktur, auch bestimmte funktionale Eigenschaften vermitteln.

Die erfindungsgemäße Zusammensetzung wird in den kosmetischen Mitteln als Perlglanzmittel vorzugsweise in einer Menge von mindestens 0,1 Gew.-%, bezogen auf das kosmetische Mittel, verwendet.

Bevorzugt wird die erfindungsgemäße Zusammensetzung in dem kosmetischen Mittel in einer Menge von 0,1 bis 12 Gew.-%, weiter bevorzugt 0,5 bis 6 Gew.-%, noch weiter bevorzugt 0,75 bis 3,5 Gew.-%, jeweils bezogen auf das kosmetische Mittel, insbesondere tensidische kosmetische Mittel, verwendet.

### Ausführungsbeispiele:

Es wurden die im Folgenden beschriebenen Untersuchungen zu den Eigenschaften der Isosorbid-Derivate vorgenommen. Soweit Inhaltstoffe genannt werden, wurde die INCI-Nomenklatur angewendet.

Dabei wurde der Perlglanz optisch durch Vergleich der Perlglanzkonzentrate der erfindungsgemäßen Formulierungen 1, 2 und 3 mit einem Standard-Perlglanzmittel (EGDS = Cutina® AGS; Vergleichsformulierungen 1 bis 3) beurteilt und auf einer Skala von 0 bis 2 bewertet (0 = kein Perlglanz, 1 = Perlglanz vergleichbar mit Standard, 2 = Perlglanz besser als Standard).

### Zusammensetzung aus Isosorbiddiester, Isosorbidmonoester und Fettsäure mit entsprechender C-Kettenverteilung zwischen C₁₆ und C₁₈:

| | C-Kettenverteilung C₁₆ und C₁₈ | | GC wt% | | |
|---|---|---|---|---|---|
| | % C16 | % C18 | Monoester | Diester | Fettsäure |
| Probe 1 | 2 | 98 | 6 | 86 | 8 |
| Probe 2 | 30 | 70 | 3 | 89 | 8 |
| Probe 3 | 45 | 55 | 4 | 83 | 13 |

### Perlglanzkonzentrate, in welche die vorstehend genannten Mischungen aus Isosorbiddiester, Isosorbidmonoester und Fettsäure (als Bestandteil I) eingesetzt wurden:

| | Formulierungen | | | | | |
|---|---|---|---|---|---|---|
| | Erfindungsgemäße Formulierung 1 | Vergleich zur erfindungsgemäßen Formulierung 1 | Erfindungsgemäße Formulierung 2 | Vergleich zur erfindungsgemäßen Formulierung 2 | Erfindungsgemäße Formulierung 3 | Vergleich zur erfindungsgemäßen Formulierung 3 |
| | Menge (Gew.-%) | | | | | |
| Erfindungsgemäße Testsubstanz (Isosorbiddiester, Isosorbidmonoester, Fettsäure) | 21,4 | | 20 | | 19,5 | |
| Cutina® AGS (Ethylenglykoldistearat) (Vergleich) | | 21,4 | | 20 | | 19,5 |
| Comperlan® 100 (INCI: Cocamide MEA) | | | | | | |
| Monomuls® 90-O 18 (INCI: Glyceryl Oleate) | 1,9 | 1,9 | | | | |
| Cutina® GMS-V (INCI: Glyceryl Stearate) | 1,5 | 1,5 | | | | |
| Plantacare® 1200 (INCI: Lauryl Glucoside, 51.5 %ig) | 15 | 15 | 37 | 37 | 36,5 | 36,5 |
| Glucopon® 215 CSUP (INCI: Decyl glucoside, 64 %ig) | 8,7 | 8,7 | 13,8 | 13,8 | 3,1 | 3,1 |
| Texapon® N70 (INCI: Sodium Laureth Sulfate 70%ig) | | | | | | |
| Glycerin (99,5 %) | | | 15,6 | 15,6 | | |
| Benzoesäure | 0,5 | 0,5 | | | 0,4 | 0,4 |
| Zitronensäure (50 %ig) | 2,3 | 2,3 | 9,8 | 9,8 | 3,9 | 3,9 |
| Wasser | Auf 100 | Auf 100 | Auf 100 | Auf 100 | Auf 100 | Auf 100 |
| pH | 4-5 | | 4-5 | 4-5 | 4-5 | 4-5 |

Die Perlglanzkonzentrate wurden dann in wässrige Haarshampooformulierungen eingebracht durch Vermischen der folgenden Bestandteile bei 25 °C:
4 Gew.-% der Perlglanzkonzentrate,
15 Gew.-% Texapon® N 70 (Kokosfettalkohol+2EO-sulfat-Natriumsalz),
3 Gew.-% Dehyton® PK45 (Kokosfettsäurebetain, zwitterionisches Tensid),
1,5 Gew.-% Natriumchlorid und
1,5 Gew.-% Plantacare® 8/18 (= Kokosalkylglucosid; nichtionisches Tensid)
sowie ad 100 Gew.-% Wasser und Konservierungsmittel.

### Ergebnisse der Perlglanzbewertung (jeweils in Bezug auf die entsprechenden Vergleichsformulierungen):

| | Formulierung 1 | Formulierung 2 | Formulierung 3 |
|---|---|---|---|
| Probe 1 | 2 | 2 | 2 |
| Probe 2 | 2 | 1 | 1 |
| Probe 3 | 1 | 1 | 1 |

Die Verbesserung des Perlglanzes wurde bestimmt im Vergleich mit den in oben stehender Tabelle angegebenen Vergleichsformulierungen, die eine zu dem erfindungsgemäßen Perlglanzwachs äquivalente Menge an Standard-Perlglanzmittel (EGDS = Cutina® AGS) enthalten.

## Patentansprüche

1. Zusammensetzung, umfassend
mindestens einen Perlglanzwachs als ersten Bestandteil, enthaltend einen Isosorbiddiester einer gesättigten C12-C24 Fettsäure, und
mindestens ein nichtionisches Tensid als zweiten Bestandteil.

2. Zusammensetzung nach Anspruch 1, **dadurch gekennzeichnet, dass** der Perlglanzwachs mindestens einen Isosorbiddiester einer gesättigten C₁₂-C₂₄ Fettsäure, mindestens einen Isosorbidmonoester einer gesättigten C₁₂-C₂₄ Fettsäure und mindestens eine gesättigte C₁₂-C₂₄ Fettsäure umfasst.

3. Zusammensetzung nach Anspruch 2, **dadurch gekennzeichnet, dass** der Isosorbiddiester in der Zusammensetzung in einer Menge von mindestens 70 Gew.-%, bezogen auf den Perlglanzwachs, enthaltend ist.

4. Zusammensetzung nach einem der Ansprüche 2 oder 3, **dadurch gekennzeichnet, dass** in der Zusammensetzung das Gewichtsverhältnis von Isosorbiddiester zu Isosorbidmonoester mindestens 4 :1 ist.

5. Zusammensetzung nach einem der Ansprüche 1 bis 4, **dadurch gekennzeichnet, dass** die Zusammensetzung
- Isosorbiddistearat, Isosorbiddipalmitat und Isosorbidpalmitatstearat als Isosorbiddiester,
- Isosorbidmonostearat und Isosorbidmonopalmitat als Isosorbidmonoester, und
- Stearinsäure und Palmitinsäure als Fettsäure umfasst.

6. Zusammensetzung nach Anspruch 5, **dadurch gekennzeichnet, dass** in der Zusammensetzung das Gewichtsverhältnis von Isosorbiddipalmitat zu Isosorbiddistearat 45 : 55 bis 1 : 99 beträgt und/oder das Gewichtsverhältnis von Isosorbidmonopalmitat zu Isosorbidmonostearat 45 : 55 bis 1 : 99 beträgt und/oder das Gewichtsverhältnis von Palmitinsäure zu Stearinsäure in der Zusammensetzung 45 : 55 bis 1 : 99 beträgt.

7. Zusammensetzung nach einem der Ansprüche 1 bis 6, **dadurch gekennzeichnet, dass** der Gehalt an Fettsäure in der Zusammensetzung 1 bis 20 Gew.-%, bezogen auf den Perlglanzwachs, beträgt.

8. Zusammensetzung nach einem der Ansprüche 1 bis 7, **dadurch gekennzeichnet, dass** das nichtionische Tensid mindestens ein Alkylpolyglykosid umfasst.

9. Zusammensetzung nach einem der Ansprüche 1 bis 8, **dadurch gekennzeichnet, dass** die Zusammensetzung den Perlglanzwachs als ersten Bestandteil in einer Menge von 10 bis 40 Gew.-%, bezogen auf die Zusammensetzung, umfasst.

10. Zusammensetzung nach einem der Ansprüche 1 bis 9, **dadurch gekennzeichnet, dass** die Zusammensetzung das nichtionische Tensid als zweiten Bestandteil in einer Menge von 5 bis 40 Gew.-%, bezogen auf die Zusammensetzung, umfasst.

11. Zusammensetzung nach einem der Ansprüche 1 bis 10, **dadurch gekennzeichnet, dass** die Zusammensetzung
(a) 10 bis 40 Gew.-% Perlglanzwachs;
(b) 5 bis 40 Gew.-% nichtionisches Tensid;
(c) 0 bis 40 Gew.-% Polyol; und
(d) ad 100 Gew.-% Wasser
enthält.

12. Verfahren zur Herstellung einer Zusammensetzung gemäß einem der Ansprüche 1 bis 11, **gekennzeichnet durch** die Verfahrensschritte
(a) des Veresterns von Isosorbid mit mindestens einer gesättigten C₁₂-C₂₄ Fettsäure unter Erhalt eines Veresterungsprodukts als ersten Bestandteil und
(b) des Mischens des aus Verfahrensschritt (a) stammenden Veresterungsprodukts mit dem zweiten Bestandteil, enthaltend mindestens ein nichtionisches Tensid.

13. Verwendung der Zusammensetzung gemäß einem der Ansprüche 1 bis 11 als Perlglanzkonzentrat.

14. Verwendung nach Anspruch 13 zur Herstellung von wässrigen, tensidischen Zubereitungen mit Perlglanzeffekt.

15. Wässrige, tensidische Zubereitung mit Perlglanzeffekt, enthaltend 0,2 bis 10 Gew.-% Perlglanzkonzentrat nach einem der Ansprüche 1 bis 11.

## Claims

1. A composition comprising
at least one pearlizing wax as first constituent, comprising an isosorbide diester of a saturated C12-C24 fatty acid, and
at least one non-ionic surfactant as second constituent.

2. The composition as claimed in claim 1, wherein the pearlizing wax comprises at least one isosorbide diester of a saturated C₁₂-C₂₄ fatty acid, at least one isosorbide monoester of a saturated C₁₂-C₂₄ fatty acid and at least one saturated C₁₂-C₂₄ fatty acid.

3. The composition as claimed in claim 2, wherein the isosorbide diester is present in the composition in an amount of at least 70% by weight, based on the pearlizing wax.

4. The composition as claimed in either of claims 2 or 3, wherein the ratio by weight of isosorbide diester to isosorbide monoester in the composition is at least 4:1.

5. The composition as claimed in any of claims 1 to 4, wherein the composition comprises
- isosorbide distearate, isosorbide dipalmitate and isosorbide palmitate stearate as isosorbide diester,
- isosorbide monostearate and isosorbide monopalmitate as isosorbide monoester, and
- stearic acid and palmitic acid as fatty acid.

6. The composition as claimed in claim 5, wherein the ratio by weight of isosorbide dipalmitate to isosorbide distearate in the composition is from 45: 55 to 1: 99 and/or the ratio by weight of isosorbide monopalmitate to isosorbide monostearate is from 45: 55 to 1: 99 and/or the ratio by weight of palmitic acid to stearic acid in the composition is from 45: 55 to 1: 99.

7. The composition as claimed in any of claims 1 to 6, wherein the content of fatty acid in the composition is 1 to 20% by weight, based on the pearlizing wax.

8. The composition as claimed in any of claims 1 to 7, wherein the non-ionic surfactant comprises at least one alkyl polyglycoside.

9. The composition as claimed in any of claims 1 to 8, wherein the composition comprises the pearlizing wax as first constituent in an amount of 10 to 40% by weight, based on the composition.

10. The composition as claimed in any of claims 1 to 9, wherein the composition comprises the non-ionic surfactant as second constituent in an amount of 5 to 40% by weight, based on the composition.

11. The composition as claimed in any of claims 1 to 10, wherein the composition comprises
(a) 10 to 40% by weight pearlizing wax;
(b) 5 to 40% by weight non-ionic surfactant;
(c) 0 to 40% by weight polyol; and
(d) up to 100% by weight water.

12. A method for preparing a composition as claimed in any of claims 1 to 11, **characterized by** the method steps of
(a) esterifying isosorbide with at least one saturated C₁₂-C₂₄ fatty acid to obtain an esterification product as first constituent and
(b) mixing the esterification product originating from method step (a) with the second constituent comprising at least one non-ionic surfactant.

13. The use of the composition as claimed in any of claims 1 to 11 as pearlescent concentrate.

14. The use as claimed in claim 13 for preparing aqueous surface-active preparations with pearlescent effect.

15. An aqueous surfactant-containing preparation with pearlescent effect comprising 0.2 to 10% by weight pearlescent concentrate as claimed in any of claims 1 to 11.

## Revendications

1. Composition, comprenant :
au moins une cire nacrée en tant que premier constituant, contenant un diester d'isosorbide d'un acide gras saturé en C₁₂-C₂₄, et
au moins un tensioactif non ionique en tant que deuxième constituant.

2. Composition selon la revendication 1, **caractérisée en ce que** la cire nacrée comprend au moins un diester d'isosorbide d'un acide gras saturé en C₁₂-C₂₄, au moins un monoester d'isosorbide d'un acide gras saturé en C₁₂-C₂₄ et au moins un acide gras saturé en C₁₂-C₂₄.

3. Composition selon la revendication 2, **caractérisée en ce que** le diester d'isosorbide est contenu dans la composition en une quantité d'au moins 70 % en poids, par rapport à la cire nacrée.

4. Composition selon l'une quelconque des revendications 2 ou 3, **caractérisée en ce que** le rapport en poids entre le diester d'isosorbide et le monoester d'isosorbide dans la composition est d'au moins 4:1.

5. Composition selon l'une quelconque des revendications 1 à 4, **caractérisée en ce que** la composition comprend :
- du distéarate d'isosorbide, du dipalmitate d'isosorbide et du palmitate-stéarate d'isosorbide en tant que diesters d'isosorbide,
- du monostéarate d'isosorbide et du monopalmitate d'isosorbide en tant que monoesters d'isosorbide, et
- de l'acide stéarique et de l'acide palmitique en tant qu'acides gras.

6. Composition selon la revendication 5, **caractérisée en ce que**, dans la composition, le rapport en poids entre le dipalmitate d'isosorbide et le distéarate d'isosorbide est de 45:55 à 1:99, et/ou le rapport en poids entre le monopalmitate d'isosorbide et le monostéarate d'isosorbide est de 45:55 à 1:99, et/ou le rapport en poids entre l'acide palmitique et l'acide stéarique dans la composition est de 45:55 à 1:99.

7. Composition selon l'une quelconque des revendications 1 à 6, **caractérisée en ce que** la teneur en acide gras dans la composition est de 1 à 20 % en poids, par rapport à la cire nacrée.

8. Composition selon l'une quelconque des revendications 1 à 7, **caractérisée en ce que** le tensioactif non ionique comprend au moins un alkylpolyglycoside.

9. Composition selon l'une quelconque des revendications 1 à 8, **caractérisée en ce que** la composition comprend la cire nacrée en tant que premier constituant en une quantité de 10 à 40 % en poids, par rapport à la composition.

10. Composition selon l'une quelconque des revendications 1 à 9, **caractérisée en ce que** la composition comprend le tensioactif non ionique en tant que deuxième constituant en une quantité de 5 à 40 % en poids, par rapport à la composition.

11. Composition selon l'une quelconque des revendications 1 à 10, **caractérisée en ce que** la composition contient :
(a) 10 à 40 % en poids de cire nacrée ;
(b) 5 à 40 % en poids de tensioactif non ionique ;
(c) 0 à 40 % en poids de polyol ; et
(d) jusqu'à 100 % en poids d'eau.

12. Procédé de fabrication d'une composition selon l'une quelconque des revendications 1 à 11, **caractérisé par** les étapes de procédé suivantes :
(a) l'estérification d'isosorbide avec au moins un acide gras saturé en C₁₂-C₂₄ pour obtenir un produit d'estérification en tant que premier constituant, et
(b) le mélange du produit d'estérification issu de l'étape de procédé (a) avec le deuxième constituant, contenant au moins un tensioactif non ionique.

13. Utilisation de la composition selon l'une quelconque des revendications 1 à 11 en tant que concentré nacrant.

14. Utilisation selon la revendication 13 pour la fabrication de préparations tensioactives aqueuses à effet nacrant.

15. Préparation tensioactive aqueuse à effet nacrant, contenant 0,2 à 10 % en poids de concentré nacrant selon l'une quelconque des revendications 1 à 11.
